# EUROPEAN PATENT APPLICATION

(11) **EP 2 578 693 A1**
(43) Date of publication of application: **10.04.2013**
(21) Application number: 11184372.8
(22) Date of filing: 07.10.2011
(51) Int. Cl.: C12Q 1/04, G01N 33/569

(54) **Method for detecting bacteria**

(71) Applicant: Koninklijk Instituut voor de Tropen, 1092 AD Amsterdam (NL); Microdish, 3584 CH Utrecht (NL)
(72) Inventor: Anthony, Richard, 1075 TS Amsterdam (NL); den Hertog, Alice, 3452 BS Vleuten (NL); Ingham, Colin John, 3515 GJ Utrecht (NL); ter Maat, Jurjen, 2317 NW Leiden (NL)
(74) Representative: Van Someren, Petronella F. H. M.

(57) **Abstract**

The invention relates to a method for the detection of bacteria, in particular *Mycobacteria* and related organisms, more in particular *Mycobacterium tuberculosis,* comprising the steps of culturing the bacteria and monitoring bacterial growth by automated imaging. For this the bacteria are preferably cultured in the presence of an optically detectable probe, in particular a luminescent or luminogenic probe, more in particular a fluorescent or fluorogenic probe, a phosphorescent or phosphorogenic probe, or a chemiluminescent or chemiluminogenic probe.

## Description

The present invention relates to a method for the detection of bacteria, in particular *Mycobacteria* and related organisms, more in particular *Mycobacterium tuberculosis.*

*Mycobacterium tuberculosis* is the causative agent of tuberculosis (TB). Tuberculosis is an often deadly infectious disease. It usually attacks the lungs but can also affect other parts of the body. It is spread through the air, by coughing, sneezing or spitting. Symptoms are very variable but classically include a chronic cough with blood-tinged sputum, fever, night sweats, and weight loss.

Diagnosis relies on radiology, tuberculin skin tests, blood tests, as well as microscopic examination but culture also remains a cornerstone of tuberculosis diagnostics and surveillance. TB culture is very labour intensive and, as the doubling time of M. *tuberculosis* is 18 hours or more, requires many days or weeks to produce a result. Furthermore, it requires a laboratory with strict biosafety regulations since for identification of the bacteria the culture sample has to be opened and there is a risk of exposure of lab workers and of cross-contamination of samples.

A need therefore exists for automatic reading of the cultures. A crucial step in this is, however, the correct identification of the culture, to answer whether any detected growth is TB or not.

It is thus the object of the present invention to provide a method for the detection of microbial growth, in particular growth of *Mycobacterium tuberculosis,* in a closed setting, i.e. without the need to open the culture sample, while maintaining the accuracy of the test result.

The invention thus relates to a method for the detection of bacteria, in particular *Mycobacteria* and related organisms, more in particular *Mycobacterium tuberculosis,* comprising the steps of culturing the bacteria and monitoring bacterial growth by automated imaging. In one embodiment, the bacteria are cultured in the presence of an optically detectable probe, in particular a fluorescent probe. In one embodiment, the bacterial growth is monitored based on rate of change in colony surface area by sequential imaging.

According to the invention the detection of microcolonies in broth (in the well-known microscopic-observation drug-susceptibility (MODS) method) or on agar, is automated by sequential imaging and/or the incorporation of an optically detectable, in particular a luminescent or luminogenic, such as a fluorescent, fluorogenic, phosphorescent, phosporogenic, chemiluminescent or chemiluminogenic, probe to the growth media or a compound that can be converted into a luminescent or luminogenic probe, more in particular a compound that can be converted into a fluorescent, fluorogenic, phosphorescent, phosphorogenic, chemiluminescent or chemiluminogenic probe.

Alternatively, the luminescent or luminogenic probes may be pre-incorporated into, or coated/bound onto the surface of a solid support. Luminescence can be monitored from outside the culture thus avoiding health hazards for the lab workers.

The invention thus relates to a method for the detection of microbial growth, in particular growth of *Mycobacterium tuberculosis,* comprising the steps of bacterial culturing, growth monitoring and data analysis, in which the monitoring of growth can be performed directly using bright field imaging, or luminescence monitoring when using a luminescent probe, where the luminescence generated is indicative for the presence of the bacteria. Luminescence may for example be fluorescence, phosphorescence, chemiluminescence or any other form.

In one embodiment of the invention bacterial growth is directly monitored e.g. using bright field imaging.

"Optically detectable probe" as used herein is intended to refer to any probe or label that can be detected by optical inspection. "Optical inspection" means any type of observation involving light. Optical inspection may for example include but is not limited to visual inspection by the human eye or through a microscope, by scanning with a laser or any other form of imaging.

The probe is preferably a luminescent or luminogenic probe, in particular a fluorescent or fluorogenic compound, a phosphorescent or phosphorogenic probe, or a chemiluminescent or chemiluminogenic probe, that is capable of specifically or semi-specifically interacting with *Mycobacteria,* in particular *Mycobacterium tuberculosis,* or enzymes produced by these species, resulting in an increased luminescence level, or altered ratio of luminescence at two or more wavelengths, which is indicative for the presence of the bacteria to be detected. The luminescence is for example fluorescence or any other form, such as phosphorescence or chemiluminescence.

In one embodiment of the invention a probe is used, that is an enzyme substrate that upon conversion by an enzyme becomes luminescent, in particular fluorescent, phosphorescent or chemiluminscent. In this embodiment, it is important that the enzyme substrate is specific or semi-specific for the *Mycobacteria,* in particular *Mycobacterium tuberculosis.*

In one embodiment the probe is a substrate for the enzyme nitrate reductase. Nitrate reductase activity is a phenotypic trait that is capable of differentiating between *Mycobacterium tuberculosis* and *Mycobacterium bovis* and is thus a suitable enzyme to identify *Mycobacterium tuberculosis.*

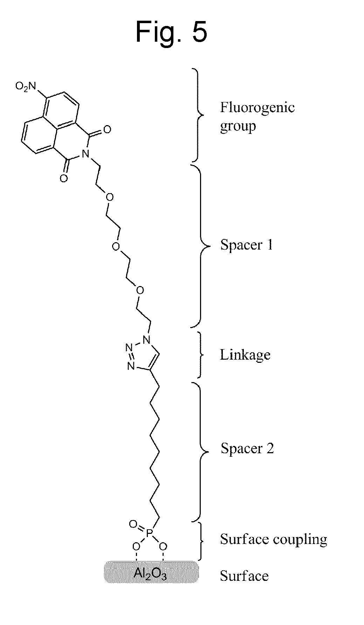

A suitable fluorogenic substrate for nitrate reductase is 6-chloro-9-nitro-5-oxo-5H-benzo(a)phenoxazine (also known as CNOB). Other suitable fluorogenic substrates include 4-nitro-1,8-naphthalimide and its N-coupled derivatives **(****Figure 5****).** The 4-nitro-1,8-naphthalimide may be substituted at its 3 and/or 6 position with hydrogen, hydroxyl, sulfonic acid, sulfonate, methoxy, chlorine or bromine. In addition, it may be substituted at its 5 position with hydrogen, bromine, chlorine or nitro. The N-coupled group may consist of, but is not limited to: hydrogen, methyl, ethyl, butyl, 2-hydroxyethyl, 3-aminopropyl, hexanoic acid and hydrazinecarboxamide.

The 4-nitro-1,8-naphthalimide-based substrate may also be N-coupled to a spacer that is coupled to a functional group which can be conjugated to a surface **(****Figure 5****).** Spacers include linear or branched alkyl (CH₂)ₙ or oligoethylene oxide (CH₂CH₂O)ₙ where n can range from 1 to 20, and linear combinations hereof. Spacers may contain linkage groups, such as amide (-C(=O)NH-), ester (-C(=O)O-) and 1,2,3-triazole. Surface-conjugating groups may include trialkoxysilane, trichlorosilane, phosphonic acid, ethylene, acetylene and thiol.

In one embodiment the probe becomes luminescent, in particular fluorescent, phospohorescent or chemiluminescent, upon incorporation in or attachment to the *Mycobacteria.* The cell wall of *Mycobacteria* is very distinct from that of other bacteria. They have a waxy coating on the cell surface that primarily consists of mycolic acid. Since this cell wall is typical for *Mycobacteria,* selection could be made on the basis of this.

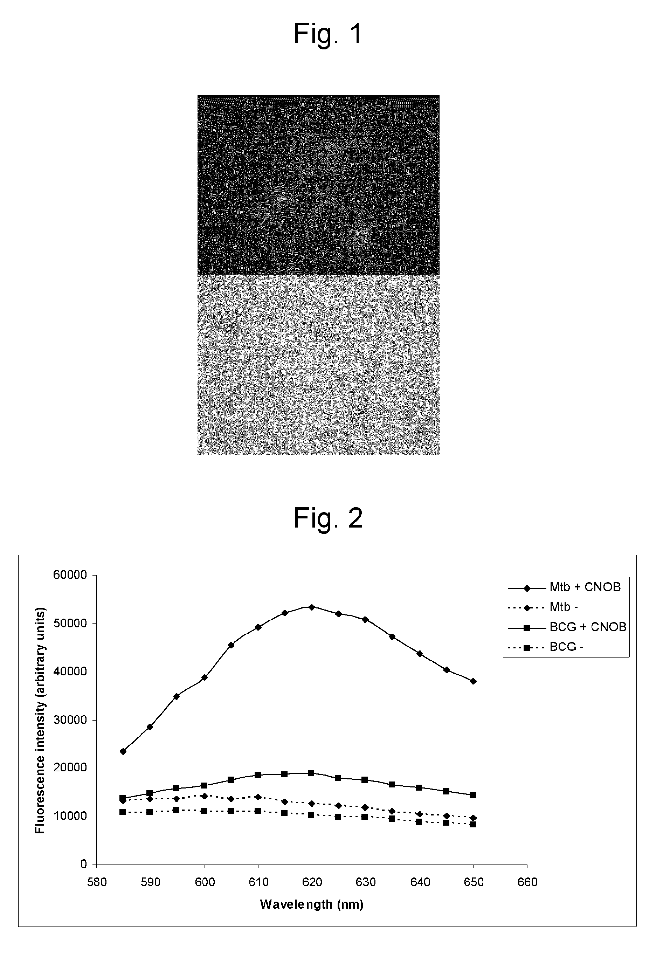

As the luminescent or luminogenic probes are to be incorporated in the growth medium and are thus in contact with the bacteria to be detected, they should be non-toxic to the bacteria cultured, and be soluble and stable in the medium. A selection of the probes described herein, in particular CNOB, meet these criteria **(****Figure 2****).**

In another embodiment, the fluorogenic probe is immobilized to a solid support, and becomes fluorescent in contact with or in the vicinity of *Mycobacteria* or from the release of an enzyme from damaged cells. Because of the immobilization, many mechanisms of toxicity and limitations in solubility do not apply to probes used in this fashion. Immobilization may result in retention of the substrate on a surface after enzymatic conversion or release.

Alternatively, release products may be captured onto another surface or by M. *tuberculosis* cells or subjected to further chemical reaction or enzymatic conversion.

In another approach, the slow release of the luminogenic substrate from a surface or particle or other agent may be used to optimise dosage of cells whilst minimising any toxicity. In the event of release of the substrate this may be triggered by an event or cellular activity or a controlled release over time.

The invention can also be used with other types of probes provided they are or can be made visible from outside the culture.

The invention can also be used with multiple probes.

According to the invention, the probes described above can be applied both for growth detection and colony identification. The probe can simply be added to the media upon preparation thereof, or can be immobilized to the solid substrate.

In one embodiment, bacteria are inoculated on a flat surface, in particular on solid medium or a porous support, in particular a flat porous support. The surface is monitored for microbial growth, e.g. using bright field microscopy using an automated microscope. Monitoring comprises a series of optical inspections with comparison over time.

In one embodiment, a probe that is semi-specific for M. *tuberculosis* is incorporated into the growth media and used to stain actively growing colonies on a flat surface, in particular a solid medium or a flat porous support. In another embodiment, the probe that is semi-specific for M. *tuberculosis* is immobilized to the solid support. In both cases, the luminescence, such as fluorescence, of the surface is periodically microscopically monitored for growth using an automated microscope at one or more specific wavelengths.

The analysis of data collected as mentioned above consists of first combining the set of photos made of the solid support from each sample into one large image (stitching), and aligning these images to images from the same sample at other time points so that every individual colony has a similar XY position at each analysed time point. Next, all objects that are potentially colonies are extracted from the images and based on their sizes, shapes and XY positions, and on the images at previous time points, identified as colony.

Sequential images as obtained above simplify automated image analysis, allowing the identification of growing colonies and an estimate of their growth rate to be obtained. In this way colonies can be identified on the basis of their growth rate and where relevant, interaction with the probe. As culture is performed on solid porous supports, these can also be moved to a selective medium, e.g. containing antibiotics, to measure the effect of that medium on bacterial growth.

The present invention is illustrated in the Examples that follow and that are not intended to limit the invention in any way. In the Examples reference is made to the following figures:
**Figure 1****:** Fluorescence and bright field image of a microscopic field containing colonies of Mycobacteria grown on agar in the presence of 1µg/ml of the enzyme substrate CNOB.
**Figure 2****:** Fluorescence emission spectrum of Mycobacterium tuberculosis colonies and other *Mycobacterium* colonies grown on agar containing a nitrate reductase substrate
**Figure 3****:** Growth of mycobacterial colonies in the presence of CNOB.
**Figure 4****:** Example of the detection of colony growth and growth rate measurements
**Figure 5****:** Example of a 4-nitro-1,8-naphthalimide-based probe that is coupled to an aluminium oxide surface.

### EXAMPLES

### EXAMPLE 1

### Detection of Mycobacterium tuberculosis with an enzyme substrate probe

*Mycobacteria* were grown to colonies on agar in the presence of 1µg/ml of the enzyme substrate CNOB. A fluorescence and bright field image of a microscopic field containing such colonies is shown in **Figure 1****.**

This example demonstrates the possibility to use a probe incorporated in growth media to detect Mycobacterial colonies.

### EXAMPLE 2

### Differential fluorimetric detection of Mycobacterium tuberculosis with an enzyme substrate probe

*Mycobacteria* were grown on agar in the presence of 1µg/ml of the enzyme substrate CNOB. The fluorescence spectrum was measured in a fluorimeter. The example demonstrates an increase in fluorescence intensity for M. *tuberculosis* in the presence of CNOB (Mtb + CNOB) not seen with another *Mycobacterium* with a lower level of nitrate reductase activity (BCG + CNOB), nor in the absence of the probe.

This example demonstrates the possibility to use a probe incorporated in growth media to detect enzyme activity that is sufficiently specific for *Mycobacterium tuberculosis* to facilitate diagnosis.

### EXAMPLE 3

### Growth rate in the presence of concentrations of the probe CNOB

This example demonstrates that no reduction of growth of *Mycobacteria* on agar is detected when CNOB is incorporated in the growth medium at concentrations up to at least 1µg/ml). The growth rate of Mycobacterial colonies was determined based on the surface area of colonies calculated from images made using an automated microscope at sequential time points. Combining the growth rate with fluorescence detection enables more specific identification of *Mycobacteria* or M. *tuberculosis.*

### EXAMPLE 4

### Detection of the growth rate of Mycobacterial microcolonies

This example demonstrates the detection and measurement of microcolony growth on solid supports in experiments during which the supports are moved onto different media.

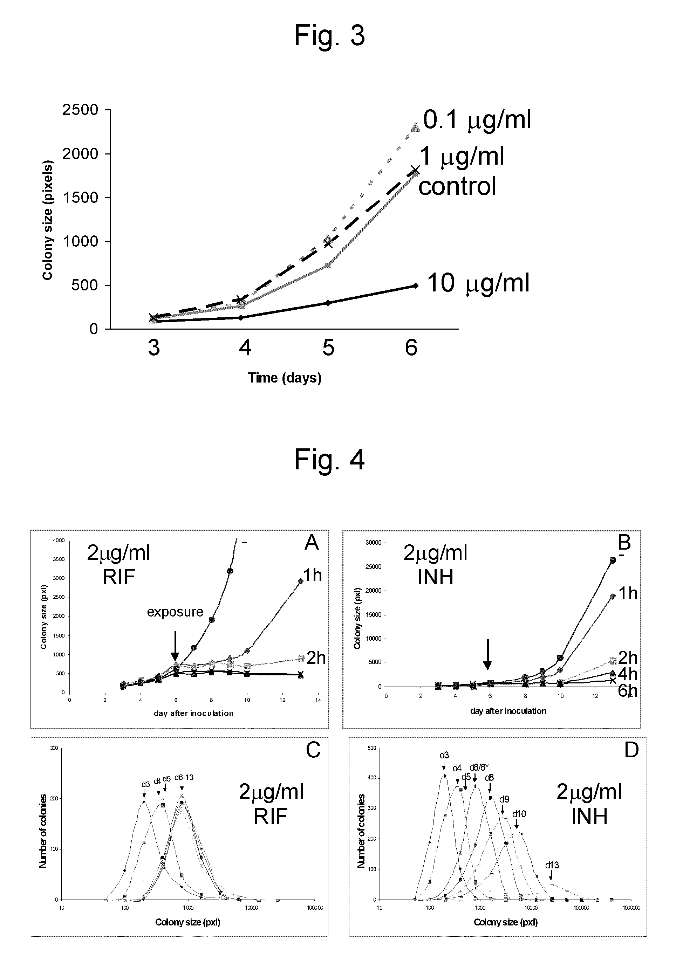

The growth rates are averages of sizes of individual Mycobacterial microcolonies detected in the serial images. The sizes, or surface areas, of colonies were calculated from images made using an automated microscope at sequential time points. In this particular example, 6 day old microcolonies were exposed for 1, 2, 4 or 6 hours with 2µg/ml rifampicin (RIF, **Figure 4A****)** or 2 µg/ml isoniazid (INH, **Figure 4B****),** after which culture and colony imaging was continued.

**Figures 4C** and **4D** show the size distributions of microcolonies at the sequential time points of experiments where growth is inhibited after a short exposure with RIF **(****Figure 4C****)** or growth continues after a short exposure with INH **(****Figure 4D****).**

## Claims

1. Method for the detection of bacteria, in particular *Mycobacteria* and related organisms, more in particular *Mycobacterium tuberculosis,* comprising the steps of culturing the bacteria and monitoring bacterial growth by automated imaging.

2. Method as claimed in claim 1, wherein the bacteria are cultured in the presence of an optically detectable probe, in particular a luminescent or luminogenic probe, more in particular a fluorescent or fluorogenic probe, a phosphorescent or phosphorogenic probe, or a chemiluminescent or chemiluminogenic probe or a compound that can be converted into a luminescent or luminogenic probe, more in particular a compound that can be converted into a fluorescent or fluorogenic probe, into a phosphorescent or phosphorogenic probe, or into a chemiluminescent or chemiluminogenic probe.

3. Method as claimed in claim 2, wherein the bacterial growth is monitored based on the optical detection of the probe, in particular the fluorescence, phosphorescence or chemiluminescence of the probe, by automated imaging.

4. Method as claimed in any one of the claims 1-3, wherein monitoring of bacterial growth is based on rate of change in colony surface area by sequential imaging.

5. Method as claimed in any one of the claims 2-4, wherein monitoring of the optically detectable probe and monitoring of the rate of bacterial growth are combined.

6. Method as claimed in any one of the claims 1-5, wherein the bacteria grow as colonies on a solid medium.

7. Method as claimed in any one of the claims 1-6, wherein monitoring is further used for identification of the bacteria.

8. Method as claimed in any one of the claims 1-7, wherein the bacteria grow as colonies on a porous support, in particular a flat porous support.

9. Method as claimed in claim 6, 7 or 8, wherein the probe is coated on, incorporated in or immobilized to the solid medium or porous support.

10. Method as claimed in any one of the claims 2-3 or 5-9, wherein the probe is an enzyme substrate that upon conversion by the enzyme becomes fluorescent, phosphorescent or chemiluminescent.

11. Method as claimed in claim 10, wherein the probe is a substrate for the enzyme nitrate reductase.

12. Method as claimed in any one of the claims 2-11, wherein the probe is 6-chloro-9-nitro-5-oxo-5H-benzo(a)phenoxazine.

13. Method as claimed in any one of the claims 2-11, wherein the probe is 4-nitro-1,8-naphthalimide or a derivative hereof.

14. Method as claimed in claim 4 or 5, wherein sequential imaging is performed at one or more wavelengths.

15. Method as claimed in any of the claims 1-14, wherein culture conditions such as atmosphere, incubation conditions and medium components are adapted during the course of microcolony culturing.
